# EUROPEAN PATENT APPLICATION

(11) **EP 4 575 501 A1**
(43) Date of publication of application: **25.06.2025**
(21) Application number: 23854897.8
(22) Date of filing: 16.08.2023
(51) Int. Cl.: G01N 33/531, G01N 33/543, G01N 33/545

(54) **IMMUNOASSAY METHOD AND REAGENT**

(30) Priority: 18.08.2022 JP 2022130544
(71) Applicant: Denka Company Limited, Tokyo 103-8338 (JP)
(72) Inventor: YANAGITA Tomoyo, Tokyo 103-8338 (JP); AKAO Kento, Tokyo 103-8338 (JP); KANDA Takaaki, Tokyo 103-8338 (JP)
(74) Representative: Hoffmann Eitle
(86) International application number: PCT/JP2023/029553
(87) International publication number: WO 2024/038863

(57) **Abstract**

To provide an assay method and a reagent which improve the long-term stability of measurement values of a biological substance. Provided are an assay method and reagent in which a non-ionic surfactant having a surface tension of 50 mN/m at a concentration equal to or greater than a critical micelle concentration is added to a reaction liquid.

## Description

### TECHNICAL FIELD

The present invention relates to: an immunoassay method; a reagent therefor; and a method using the reagent.

### BACKGROUND

Immunoassay methods are applied in clinical tests as methods for determining quantities of antigenic substances or antibodies included a bodily fluid such as blood serum, blood plasma, urine, spinal fluid, etc., and methods adapted to automatic analyzers for biochemical testing are now becoming widespread. In recent years, in immunoassay methods for a purpose of clinical testing, a higher sensitivity has been achieved by using insoluble carriers, etc., and measurement accuracy has improved significantly. There have been many developments of reagents that make high-sensitivity measuring possible by using, for example, a technique in which two or more kinds of insoluble carrier particles with different particle sizes are used (see Patent Document 1).

However, in reagents in which a higher sensitivity brings about improved measurement accuracy, there is a high level of sensitivity, and therefore, a slight variation in an external or internal factor may have an influence on measurements. In particular, in states in which long-term stability is unsound, there have been issues wherein variations occur in measurement values.

### CITATION LIST

### PATENT LITERATURE

Patent Document 1: JP 2588174 B

### SUMMARY OF INVENTION

### TECHNICAL PROBLEM

The objective of the present invention is to provide: an immunoassay method in which the influence of interfering substances can be suppressed to improve the accuracy of the immunoassay method; and a reagent for an immunoassay method.

### SOLUTION TO PROBLEM

As a result of diligent research, the present inventors discovered that by adding at least one surfactant to a reaction liquid at a concentration such that the surface tension in an aqueous solution is 50 mN/m or more, it is possible to suppress the influence of interfering substances present in blood serum and improve the accuracy of an immunoassay method, and thus, completed the present invention.

That is, the present invention is as described below.
[1] An immunoassay method in which at least one surfactant having a surface tension in a 1.0% aqueous solution of 50 mN/m or more is added to a reaction liquid at a concentration such that the surface tension in an aqueous solution is 50 mN/m or more.
[2] The assay method of [1], wherein the assay method is an immunoagglutination method.
[3] The assay method of [2], wherein the assay method is a latex agglutination method.
[4] The assay method of any one of [1]-[3], wherein the surfactant is a non-ionic surfactant.
[5] The assay method of any one of [1]-[3], wherein the surfactant is at least one surfactant selected from the group consisting of polyoxyethylene myristyl ether, polyoxyethylene distyrenated phenyl ether, polyoxyethylene lauryl ether, polyoxyethylene cetyl ether, polyoxyethylene stearyl ether, polyoxyethylene oleyl ether, polyoxyethylene alkyl ether, polyoxyethylene octyl dodecyl ether, polyoxyethylene alkylene alkyl ether, polyoxyethylene tribenzyl phenyl ether, polyoxyethylene polyoxypropylene glycol, polyoxyethylene glyceryl ether, and polyoxyethylene diglyceryl ether.
[6] An assay reagent obtained by adding at least one surfactant having a surface tension in a 1.0% aqueous solution of 50 mN/m or more to a reaction liquid at a concentration such that the surface tension in an aqueous solution is 50 mN/m or more.
[7] The assay reagent of [6], wherein the assay reagent includes a first reagent and a second reagent.
[8] The assay reagent of [6] or [7], wherein the assay reagent is an immunoagglutination reagent.
[9] The assay reagent of [8], wherein the assay reagent is a latex agglutination reagent.
[10] The assay reagent of any one of [6]-[9], wherein the surfactant is a non-ionic surfactant.
[11] The assay reagent of any one of [6]-[9], wherein the surfactant is at least one surfactant selected from the group consisting of polyoxyethylene alkyl ether, polyoxyethylene distyrenated phenyl ether, polyoxyethylene lauryl ether, polyoxyethylene cetyl ether, polyoxyethylene stearyl ether, polyoxyethylene oleyl ether, polyoxyethylene alkyl ether, polyoxyethylene octyl dodecyl ether, polyoxyethylene alkylene alkyl ether, polyoxyethylene tribenzyl phenyl ether, polyoxyethylene polyoxypropylene glycol, polyoxyethylene glyceryl ether, and polyoxyethylene diglyceryl ether.
[12] A method for recovering a capability decrease of an immunoassay reagent during storage in an immunoassay method, the method including adding at least one surfactant having a surface tension in a 1.0% aqueous solution of 50 mN/m or more to a reaction liquid at a concentration such that the surface tension in an aqueous solution is 50 mN/m or more.
[13] The method of [12], wherein the method is an immunoagglutination method.
[14] The method of [13], wherein the method is a latex agglutination method.
[15] The method of any one of [12]-[14], wherein the surfactant is a non-ionic surfactant.
[16] The method of any one of [12]-[14], wherein the surfactant is at least one surfactant selected from the group consisting of polyoxyethylene myristyl ether, polyoxyethylene distyrenated phenyl ether, polyoxyethylene lauryl ether, polyoxyethylene cetyl ether, polyoxyethylene stearyl ether, polyoxyethylene oleyl ether, polyoxyethylene alkyl ether, polyoxyethylene octyl dodecyl ether, polyoxyethylene alkylene alkyl ether, polyoxyethylene tribenzyl phenyl ether, polyoxyethylene polyoxypropylene glycol, polyoxyethylene glyceryl ether, and polyoxyethylene diglyceryl ether.

### EFFECTS OF INVENTION

By using the immunoassay method according to the present invention, it is possible to achieve long-term stability and accurately measure a substance to be measured. Further, the long-term storage stability of a reagent can be improved by using the immunoassay reagent according to the present invention.

### BRIEF DESCRIPTION OF DRAWINGS

Fig. 1 shows results achieved by measuring a specimen using a first reagent in which polyoxyethylene distyrenated phenyl ether was added, and a second reagent before and after storage.
Fig. 2 shows results achieved by measuring a specimen using a first reagent in which polyoxyethylene alkylene alkyl ether and polyoxyethylene distyrenated phenyl ether were added, and a second reagent before and after storage.
Fig. 3 shows results achieved by measuring a specimen using a first reagent in which polyoxyethylene alkylene alkyl ether and polyoxyethylene myristyl ether were added, and a second reagent before and after storage.
Fig. 4 shows results achieved by measuring a specimen using a first reagent in which polyoxyethylene alkylene alkyl ether was added, and a second reagent before and after storage.

### DESCRIPTION OF EMBODIMENTS

The method according to the present invention is described below. It should be noted that "%" herein means mass standard (w/v%) except where specifically indicated.

The present invention is an immunoassay method in which the influence of interfering substances can be suppressed to improve the accuracy of the immunoassay method.

The present invention can be applied to an immunological assay method using any antigen-antibody reaction. That is, the present invention can, for example, be applied to an immunodiffusion method, an immunoturbidimetry method, an immunonephelometry method, a hemagglutination method, a latex method, an enzyme immunoassay method (EIA method), radioimmunoassay method (RIA method), an immunochromatography method, etc. Among the foregoing, the greatest effects are exhibited in the immunoturbidimetry method, the immunonephelometry method, and agglutination methods such as the hemagglutination method and the latex agglutination method.

An agglutination method is an immunoassay method by agglutination using a non-soluble carrier such as latex particles, bentonite, collodion, kaolin, fixed sheep erythrocytes, etc. As the insoluble carrier, latex is preferable and as latex particles, it is possible to use, for example, polystyrene latex particles, styrene-butadiene copolymer latex particles, polyvinyltoluene latex particles, etc. In an immunoassay by an agglutination method, in a case in which a measurement target is an antigen, the insoluble carrier should be sensitized with an antibody, that is, an antibody should be bound to a carrier surface, and in a case in which the measurement target is an antibody, the carrier should be sensitized with an antigen.

In the immunoassay method according to the present invention, measuring is preferably performed using an automatic analyzer. An automatic analyzer is a biochemical examination automatic analyzer which can be applied in an immunoassay method, and employs a mechanism in which a reagent is dispensed to a reaction tank via a tube using a continuous dispensing method. That is, the automatic analyzer comprises a means for disposing a reagent inside the analyzer or outside the analyzer, wherein the reagent passes through a tube and is continuously dispensed to the inside of a reaction tank provided to the analyzer. Dispensing a reagent that has passed through the tube is performed, for example, by a combination of a pump such as a peristaltic pump, or the like, and a switching valve. Examples of such a device include various commercially available products, including, for example, the automatic analyzer 7180, the automatic analyzer 7250, the automatic analyzer 7450, and the automatic analyzer 7350 which are manufactured by Hitachi, Ltd.

In a method in which measuring is carried out by using an automatic analyzer, antigens or antibodies are measured by optically capturing agglutinated clumps formed by measurement-target antibodies or measurement-target antigens binding to an antigen- or antibody-sensitized carrier in a reaction tank. That is, the degree of agglutination of the carrier particles is measured by irradiating a light in the range from visible light to near-infrared onto an agglutinated clump and detecting a change in light absorbance or a change in scattered light intensity. Examples of measurement methods at that time include a one point method, a two point method, a three point method, and a rate method, etc. The method according to the present invention can be applied to any measurement method. By dispensing a testing sample into a reaction tank, then dispensing a first reagent and a second reagent, allowing a reaction to occur, and measuring the degree of agglutination in advance, it is possible use the degree of agglutination as an index to determine the quantity of a measurement-target antigen or a measurement-target antibody in the testing sample. The order in which the first reagent and the second reagent are added is not limited but it is preferable for the first reagent to be dispensed prior to the second reagent or for the first reagent and the second reagent to be dispensed simultaneously. At that time, by creating, in advance, a standard curve associating the degree of agglutination with a testing sample concentration by using a plurality of samples in which the concentration of the measurement target is known, it is possible to calculate the concentration of the measurement target in the testing sample on the basis of the standard curve.

A buffer solution is an example of the first reagent, and a stabilizing reagent, a protecting reagent, etc., may also be included. Examples of the second reagent include the particles described above which have sensitized antibodies or antigens specifically binding to the antigen or antibody that is the measurement target. A stabilizing reagent, a protecting reagent, etc., may also be included.

The method for sensitizing the carrier with an antibody or an antigen is not particularly limited but should be in accordance with publicly-known methods. For example, an antigen or antibody may be physically adsorbed to the carrier or may be chemically bonded thereto. More specifically, for example, by mixing an antibody or antigen with the carrier and then warming and shaking for one to two hours at 30-37°C, the carrier can be sensitized with an antibody. The amount of the antigen or antibody to be sensitized on the carrier can be set, as appropriate, in accordance with the particle size of the carrier used. After the carrier is sensitized with an antibody or antigen, non-sensitized sections on the carrier surface are preferably blocked with bovine serum albumin, human serum albumin, rabbit serum albumin, or ovalbumin, etc. A carrier that has been sensitized with an antibody or antigen is preferably held as a medium dispersion liquid until the carrier is to be reacted with a testing sample. At that time, a phosphate buffer solution or a glycine buffer solution, etc., can be used as a medium. Bovine serum albumin, gelatin, gum arabic, etc., may also be added to the medium as needed. An antigen or antibody in a testing sample can be detected by reacting an antibody- or antigen-sensitized carrier prepared in the manner described above with the testing sample and determining the reactivity between the sensitized antibodies or antigens and the antigen or antibody in the testing sample from the presence or absence of agglutination or the degree thereof.

In an agglutination method using sensitized particles, detecting involves adding and mixing several tens of µL to several hundreds of µL of a physiological saline solution or a suitable buffer solution to several µL to several tens of µL of a testing sample in a suitable reaction container, incubating for several minutes, then adding several tens of µL to several hundreds of µL of sensitized particles having antibodies or antigens bound thereto, and incubating for several minutes. Next, by measuring light absorbance at a suitable measurement wavelength (for example, 570 nm), it is possible to measure turbidity due to agglutination of the sensitized particles caused by an antigen-antibody reaction. Further, several tens of µL to several hundreds of µL of a sensitized particle solution suspended in a buffer solution may be added to several µL of a testing sample. A suitable buffer solution is a buffer solution with a pH of 5.0-10 and examples thereof include phosphate buffer solutions, boric acid buffer solutions, and tris buffer solutions, etc.

The testing sample handled by the immunoassay method according to the present invention is not particularly limited but is preferably a biological sample in which the effect of the present invention, namely, suppressing the influence of interfering substances, can be exhibited significantly. In particular, bodily fluids such as blood, blood serum, blood plasma, urine, feces, saliva, tissue fluid, spinal fluid, swabs, etc., or dilutions thereof are preferred, with blood, blood serum, and blood plasma or dilutions thereof being further preferred.

The measurement target, that is, the substance to be measured, in the immunoassay method according to the present invention is not limited to a particular substance, and may be a protein, sugar, or other compound present in a biological sample, and may also be a substance with a biological origin. Further, the measurement target may be a composite of the foregoing, for example, a pathogenic microorganism such as bacteria or a virus, etc., or may be a biological substance in a living organism or a substance in the environment.

In the method according to the present invention, a surfactant is present in the reaction system at the time of the antigen-antibody reaction. In the method according to the present invention, a surfactant is used at a concentration at which the surface tension in an aqueous solution is 50 mN/m or more. Preferably, a surfactant having a surface tension in a 1.0% aqueous solution of 50 mN/m or more is used. That is, a surfactant having a surface tension in a 1.0% aqueous solution of 50 mN/m or more is to be used at a concentration at which the surface tension in an aqueous solution is 50 mN/m or more. The surfactant is also a surfactant having a surface tension of 50 mN/m or more in a concentration equal to or greater than a critical micelle concentration (cmc). When the concentration of a surfactant solution is increased, the surface tension of the surfactant solution gradually decreases and is eventually kept constant even if the concentration is increased. At that time, adsorption to an interface is saturated in the surfactant and aggregates called micelles are formed. The concentration at which micelles are formed is called the critical micelle concentration. The critical micelle concentration and the surface tension have inherent values due to the surfactant. For example, for a surfactant having a critical micelle concentration of 0.5% and a surface tension in a 0.5% aqueous solution of 50 mN/m, the surface tension in a solution with a higher concentration, for example, in a 1% solution, is 50 mN/m.

The surfactant used in the present invention may be an anionic surfactant, a cationic surfactant, a non-ionic surfactant, or an amphoteric surfactant, but is preferably a non-ionic surfactant.

Examples of a non-ionic surfactant used in the method according to the present invention include, specifically, at least one surfactant selected from the group consisting of polyoxyethylene myristyl ether, polyoxyethylene distyrenated phenyl ether, polyoxyethylene lauryl ether, polyoxyethylene cetyl ether, polyoxyethylene stearyl ether, polyoxyethylene oleyl ether, polyoxyethylene alkyl ether, polyoxyethylene octyl dodecyl ether, polyoxyethylene alkylene alkyl ether, polyoxyethylene tribenzyl phenyl ether, polyoxyethylene polyoxypropylene glycol, polyoxyethylene glyceryl ether, and polyoxyethylene diglyceryl ether, but is not limited thereto. Among the foregoing, polyoxyethylene myristyl ether or polyoxyethylene distyrenated phenyl ether is preferred.

The surfactant used in the present invention preferably has, in the reaction liquid at the time of a reaction, a concentration at which the surface tension in an aqueous solution is 50 mN/m. Specifically, said concentration is preferably approximately 0.001-10 w/v% and more preferably 0.01-1 w/v%, but is not particularly limited to the foregoing values.

The surface tension of the surfactant solution can, for example, be measured by the Young-Laplace method (curve-fitting method). The Young-Laplace method calculates surface tension by using image processing to fit the Young-Laplace equation from values of a profile shape and density differences of droplets formed by a needle tip. Measuring using the Young-Laplace method can be performed by using a contact angle meter. A DropMaster DMo-902, DMo-702m DMo-602, DMo-502, Dmo-901, DMo-701, DMo-601, or DMo-501 (product name, commercially available from Kyowa Interface Science Co., Ltd.), etc., may be used as the contact angle meter.

The surfactant may be included in any reagent solution used in an immunoassay method and may be added to the reaction liquid when measurement is performed. When there is one reagent solution used, the surfactant may be included in the reagent solution at the concentration described above, and when a plurality of reagent solutions are used, the surfactant may be included in the reagent solutions so that surfactant in the reaction liquid at the time of an antigen-antibody reaction is the concentration described above. For example, in a latex agglutination method, which is the above agglutination method that uses a first reagent and a second reagent, wherein 110 µL of the first reagent including a buffer solution is mixed with 8.8 µL of a specimen, which is a testing sample, incubated for several minutes before adding and mixing 55 µL of latex particles sensitized with an antigen, and then measuring an agglutination reaction as an amount of change in light absorbance, the surfactant should be added to the first reagent so as to be 0.05% or more and preferably 0.1% or more. Amounts to be added can be determined, as appropriate, for each surfactant.

By adding the above surfactant to the reaction liquid when performing immunoassay, it is possible to improve the stability of the immunoassay reagent and stability can be achieved without any variations in measurement values. Here, improving the stability of a immunoassay reagent includes: suppressing a capability decrease of the immunoassay reagent and improving stability by adding the above surfactant to the immunoassay reagent; and including the surfactant in the reaction liquid at the time of a reaction and recovering, to the same level as that immediately after preparation, the capability of an immunoassay reagent in which capability decreased during storage after being prepared. Here, an immunoassay reagent in which capability decreased during storage after being prepared indicates a reagent such as a latex particle, etc., having an antigen or antibody bonded thereto, wherein the reactivity of the reagent decreased during storage. For example, in a latex agglutination method, when a reagent including latex particles sensitized with an antigen or antibody is stored, the reactivity of the latex particles may decrease and measurement values may decrease. Even in such cases, measurement values can be recovered by adding a surfactant to the reaction liquid during a reaction and it is possible to obtain measurement values with a stability the same as that when a reagent is used immediately after being prepared. That is, a capability decrease in the assay reagent can be recovered.

In an agglutination method, a first reagent including the above surfactant should be mixed with a second reagent including latex particles. Further, when a first reagent that does not include a surfactant is mixed with a second reagent including latex particles, the above surfactant may be added as a third reagent.

According to the method of the present invention, it is possible to obtain measurement values of the same level as those immediately after preparation even for a reagent stored for at least one month, preferably at least two months, more preferably three months, more preferably four months, more preferably five months, more preferably at least six months, more preferably at least 12 months, more preferably at least 24 months, and more preferably at least 36 months after being prepared.

The present invention further provides an assay reagent including at least one surfactant having a surface tension in a 1.0% aqueous solution of 50 mN/m or more.

Furthermore, the present invention also includes an immunoassay kit including the assay reagent. When particles sensitized with antigens or antibodies are not included in the buffer solution composition, the immunoassay kit includes, separately, particles which are sensitized with antigens or antibodies, and may further include a positive control, a negative control, an instruction booklet, etc.

### EXAMPLES

The present invention is explained in detail using the following examples, but the present invention is not limited to the examples below.

### (1) Measuring surface tension of surfactant

An aqueous solution of each surfactant (polyoxyethylene distyrenated phenyl ether (product name "Emulgen A-500", commercially available from Kao Corporation), polyoxyethylene alkylene alkyl ether (product name "Emulgen LS-110", commercially available from Kao Corporation), polyoxyethylene myristyl ether (product name "Emulgen 4085", commercially available from Kao Corporation)) was prepared. The surface tension was measured by the Young-Laplace method using a DropMaster DMo-501 (product name, commercially available from Kyowa Interface Science Co., Ltd). Measurements were performed ten times for each concentration and the average values calculated are shown in Table 1. It is estimated that Emulgen A-500 and Emulgen 4085 reached the critical micelle concentration at a concentration of approximately 0.05%, and the surface tension of both when the critical micelle concentration was reached was 50 mN/m or more. Meanwhile, it is estimated that Emulgen LS-110 reached the critical micelle concentration at a concentration of 0.05%, and the surface tension at said concentration is 35 mN/m, and thus, less than 50 mN/m.

**[TABLE 1]**

| Surface tension of surfactant aqueous solution (unit: mN/m) | | | | | | |
|---|---|---|---|---|---|---|
| Surfactant | 0% | 0.0005% | 0.005% | 0.05% | 0.5% | 1.0% |
| Emulgen A-500 | 72.5 | 71.8 | 60.8 | 54.7 | 53.7 | 53.5 |
| Emulgen 4085 | 72.5 | 71.8 | 59.9 | 54.9 | 53.9 | 53.9 |
| Emulgen LS-110 | 72.5 | 67.9 | 49.2 | 35.3 | 34.2 | 34.0 |

### (2) Preparation of first reagent

A first reagent A was prepared by adding polyoxyethylene distyrenated phenyl ether (product name "Emulgen A-500, commercially available from Kao Corporation) to a liquid obtained by adding bovine serum albumin to a buffer solution (tris, pH 8.0) (Example 1). As Examples 2 and 3, first reagents B and C were prepared by adding polyoxyethylene alkylene alkyl ether (product name "Emulgen LS-110", commercially available from Kao Corporation) and Emulgen A-500 or polyoxyethylene myristyl ether (product name "Emulgen 4085", commercially available from Kao Corporation). As Comparative Example 1, a first reagent D was prepared by adding only Emulgen LS-110.

**[TABLE 2]**

| Example | First reagent | Additive (concentration) |
|---|---|---|
| Example 1 | A | + 0.1% polyoxyethylene distyrenated phenyl ether |
| Example 2 | B | + 0.01% polyoxyethylene alkylene alkyl ether |
| | | + 0.1% polyoxyethylene distyrenated phenyl ether |
| Example 3 | C | + 0.01% polyoxyethylene alkylene alkyl ether |
| | | + 0.1% polyoxyethylene myristyl ether |
| Comparative Example 1 | D | + 0.01% polyoxyethylene alkylene alkyl ether |

### (3) Preparation of second reagent

Using an antigen against Treponema pallidum, an assay reagent was prepared by an immunoagglutination method as described below.

Sensitized particles obtained by supporting 3.9 mg of Treponema pallidum antigens with respect to 1 mL of a polystyrene latex suspension were suspended so as to be 0.095% in a buffer solution (Bis-Tris, pH 6.0) to prepare a second reagent.

### (4) Measuring using automated analyzer

A Hitachi 7180 automatic analyzer was used to perform automatic measurement by an endpoint method.

Measurements were performed on 30 blood serum specimens using the first reagents and the second reagent described above. 110 µL of the first reagents A-D was added to 8.8 µL of a specimen solution, and the mixed solution was stirred and mixed at 37°C. After leaving to stand for five minutes, 55 µL of the second reagent was added and further stirred and mixed at 37°C. An agglutination reaction over approximately five minutes was measured as an amount of change in light absorbance, and the anti-Treponema pallidum antibody concentration in each specimen was calculated from a calibration curve created using a standard solution (HEPES buffer, bovine serum albumin, sodium chloride, anti-Treponema pallidum antibody, pH 7.4).

In order to reproduce a thermal load due to long-term storage, the second reagent was stored at 37°C. After being stored at 37°C for 21 days, a calibration curve was created using a standard solution, and the anti-Treponema pallidum antibody concentration in each specimen was calculated.

### (5) Comparison of second reagent storage stability by first reagent additive

In order to compare specimen measurement values of reagents before and after long-term storage, measurements were performed on specimens measured using: the first reagents A-D and the second reagent immediately after preparation; and the first reagents A-D and the second reagent which had been stored at 37°C for 21 days.

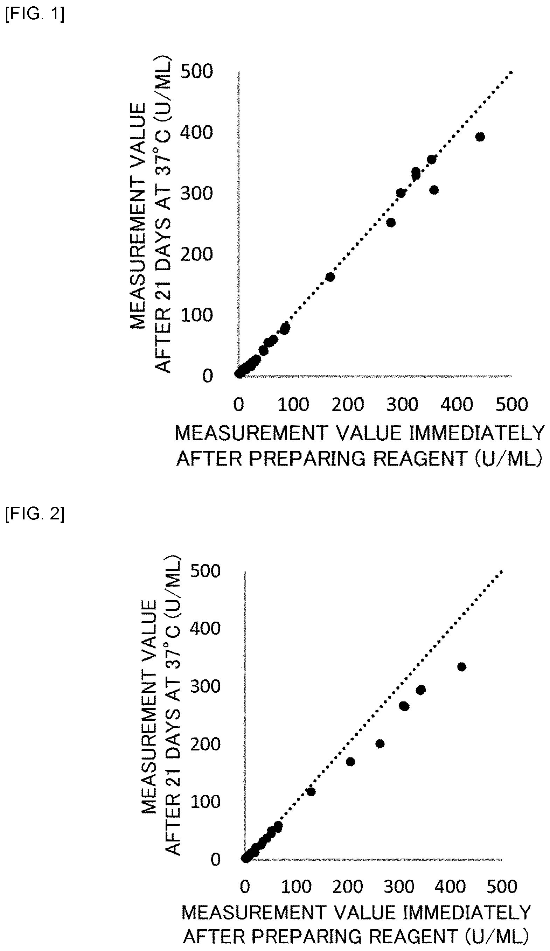

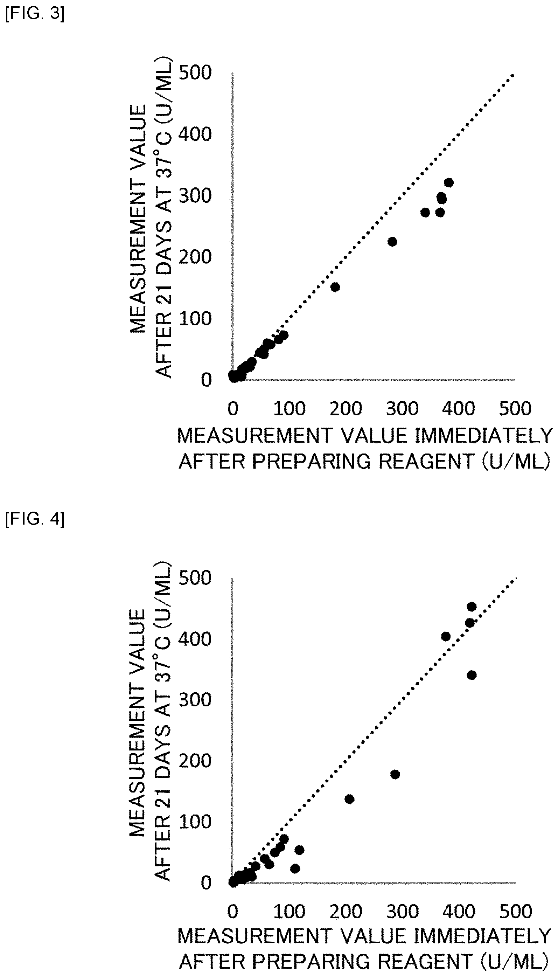

In fig. 1, which show the results for Example 1, there is little variation between specimen measurement values of the second reagent immediately after preparation and specimen measurement values of the second reagent after 21 days at 37°C, and thus, it was demonstrated that the reagent has high stability. In fig. 4, which shows the results for Comparative Example 1, a decrease in measurement values can be seen in some specimens after 21 days at 37°C. In fig. 2 and 3, which show the results for Examples 2 and 3, the decrease in specimen measurement values is small compared to fig. 4, and thus, the stability of the reagents was better than in Comparative Example 1. Table 3 shows the averages of the variation rates in 30 specimens measured using: the first reagents A-D and the second reagent immediately after being prepared; and the first reagents A-D and the second reagent after being stored at 37°C for 21 days. Examples 1-3 demonstrated stable specimen measurement values compared to Comparative Example 1. As such, it was demonstrated that by adding a surfactant having a surface tension in a 1.0% aqueous solution of 50 mN/m or more to an aqueous solution at a concentration such that the surface tension thereof is 50 mN/m or more, the stability of an immunoassay reagent improves.

**[TABLE 3]**

| Example | First reagent | Variation rate in specimen measurement values 21 days after storage at 37°C (average value, 30 specimens) |
|---|---|---|
| Example 1 | A | 100% |
| Example 2 | B | 91% |
| Example 3 | C | 105% |
| Comparative Example 1 | D | 59% |

### INDUSTRIAL APPLICABILITY

Using the method according to the present invention, it is possible to measure antibodies and antigens in a specimen.

## Claims

1. An immunoassay method in which at least one surfactant having a surface tension in a 1.0% aqueous solution of 50 mN/m or more is added to a reaction liquid at a concentration such that the surface tension in an aqueous solution is 50 mN/m or more.

2. The assay method according to claim 1, wherein the assay method is an immunoagglutination method.

3. The assay method according to claim 2, wherein the assay method is a latex agglutination method.

4. The assay method according to any one of claims 1-3, wherein the surfactant is a non-ionic surfactant.

5. The assay method according to any one of claims 1-3, wherein the surfactant is at least one surfactant selected from the group consisting of polyoxyethylene myristyl ether, polyoxyethylene distyrenated phenyl ether, polyoxyethylene lauryl ether, polyoxyethylene cetyl ether, polyoxyethylene stearyl ether, polyoxyethylene oleyl ether, polyoxyethylene alkyl ether, polyoxyethylene octyl dodecyl ether, polyoxyethylene alkylene alkyl ether, polyoxyethylene tribenzyl phenyl ether, polyoxyethylene polyoxypropylene glycol, polyoxyethylene glyceryl ether, and polyoxyethylene diglyceryl ether.

6. An assay reagent obtained by adding at least one surfactant having a surface tension in a 1.0% aqueous solution of 50 mN/m or more to a reaction liquid at a concentration such that the surface tension in an aqueous solution is 50 mN/m or more.

7. The assay reagent according to claim 6, wherein the assay reagent comprises a first reagent and a second reagent.

8. The assay reagent according to claim 6 or 7, wherein the assay reagent is an immunoagglutination reagent.

9. The assay reagent according to claim 8, wherein the assay reagent is a latex agglutination reagent.

10. The assay reagent according to any one of claims 6-9, wherein the surfactant is a non-ionic surfactant.

11. The assay reagent according to any one of claims 6-9, wherein the surfactant is at least one surfactant selected from the group consisting of polyoxyethylene myristyl ether, polyoxyethylene distyrenated phenyl ether, polyoxyethylene lauryl ether, polyoxyethylene cetyl ether, polyoxyethylene stearyl ether, polyoxyethylene oleyl ether, polyoxyethylene alkyl ether, polyoxyethylene octyl dodecyl ether, polyoxyethylene alkylene alkyl ether, polyoxyethylene tribenzyl phenyl ether, polyoxyethylene polyoxypropylene glycol, polyoxyethylene glyceryl ether, and polyoxyethylene diglyceryl ether.

12. A method for recovering a capability decrease during storage in an immunoassay reagent in an immunoassay method, the method comprising adding at least one surfactant having a surface tension in a 1.0% aqueous solution of 50 mN/m or more to a reaction liquid at a concentration such that the surface tension in an aqueous solution is 50 mN/m or more.

13. The method according to claim 12, wherein the method is an immunoagglutination method.

14. The method according to claim 13, wherein the method is a latex agglutination method.

15. The method according to any one of claims 12-14, wherein the surfactant is a non-ionic surfactant.

16. The method according to any one of claims 12-14, wherein the surfactant is at least one surfactant selected from the group consisting of polyoxyethylene myristyl ether, polyoxyethylene distyrenated phenyl ether, polyoxyethylene lauryl ether, polyoxyethylene cetyl ether, polyoxyethylene stearyl ether, polyoxyethylene oleyl ether, polyoxyethylene alkyl ether, polyoxyethylene octyl dodecyl ether, polyoxyethylene alkylene alkyl ether, polyoxyethylene tribenzyl phenyl ether, polyoxyethylene polyoxypropylene glycol, polyoxyethylene glyceryl ether, and polyoxyethylene diglyceryl ether.
